# EUROPEAN PATENT APPLICATION

(11) **EP 4 390 745 A2**
(43) Date of publication of application: **26.06.2024**
(21) Application number: 24162520.1
(22) Date of filing: 08.03.2024
(51) Int. Cl.: G06F 21/64

(54) **METHOD FOR AUTHENTICATING IMAGE DATA**

(71) Applicant: Carl Zeiss Microscopy GmbH, 07745 Jena (DE)
(72) Inventor: TZOUMAS, Stratis, 81379 Munich (DE); RHODE, Sebastian, 81379 Munich (DE)
(74) Representative: Wenzel Nemetzade Warthmüller Patentanwälte Part mbB

(57) **Abstract**

A method for authenticating image data (3) and/or metadata (5) of the image data (3) is proposed, wherein the method comprises the following steps: receiving the image data (3) and/or the metadata (5) and an encrypted first hash value of the image data (3) and/or the metadata (20), wherein the metadata (5) comprises information about the image data (3); decrypting the first hash value with a public key (11) (30), wherein the public key (11) corresponds to a private key with which the first hash value is encrypted; generating a second hash value of the image data (3) and/or the metadata (5) (40); comparing the decrypted first hash value with the second hash value (50); and generating a positive image authenticating signal (16) if the first hash value matches the second hash value or a negative image authenticating signal (16) if the first hash value does not match the second hash value (60).

## Description

The application pertains to a method for authenticating image data and a method for digitally signing processed image data.

### State of the art

Recent evolutions in machine learning, particularly through Generative Adversarial Networks (GANs) and Diffusion Models, as well as other improvements of image processing software, enable the generation of highly realistic fake images or the processing/manipulation of existing images with very realistic-looking end-result.

Consequently, it is nowadays difficult to trust the information conveyed by an image. This problem may also pose a problem in the scientific world. Image processing algorithms that employ (generative) machine learning possesses can generate fake content which may lead to ambiguity and false conclusions, especially if such data are used to support scientific hypotheses.

### Disclosure of the invention

The invention is based on the problem to provide a method for authenticating image data and/or its metadata which is reliable. It is also based on the problem to provide a method of digitally signing processed image data and/or its metadata which is reliable.

This problem is solved by a method for authenticating image data according to claim 1.

In particular, the problem is solved by a method for authenticating image data and/or metadata of the image data, wherein the method comprises the following steps: receiving the image data and/or the metadata and an encrypted first hash value of the image data and/or the metadata, wherein the metadata comprises information about the image data; decrypting the first hash value with a public key, wherein the public key corresponds to a private key with which the first hash value is encrypted; generating a second hash value of the image data and/or the metadata; comparing the decrypted first hash value with the second hash value; and generating a positive image authenticating signal if the first hash value matches the second hash value or a negative image authenticating signal if the first hash value does not match the second hash value.

One advantage hereof is that the authenticity of the image data can be tested reliably. Thus, after authenticating the image data, the image data and its content can be trusted to be an original image/original image data or to be derived from an original image/original image data. Every server/entity which has access to the public key and the image/image data and the metadata can verify the authenticity of the image data and/or the metadata. The method can be carried out on a server. All available public keys can be tested one after the other until a decrypted first hash value which matches the second hash value is found. Thus, each decrypted hash value can be compared to the computed hash value of the image data and/or the metadata. The signal generated and/or outputted can be sent back to a client which originally sent an authenticating request with the image data, the metadata of the image data and the encrypted first hash value to the server. The image/image data and the corresponding metadata can be hashed together to create a single hash value or the image/image data and the corresponding metadata can be hashed separately to create two hash values. In the latter case, both hash values can be verified independently from each other. The image data can be acquired by an image acquiring entity. The image data can comprise or be image data which has been processed.

The problem is also solved by a method of digitally signing image data according to claim 3.

In particular, the problem is solved by a method of digitally signing processed image data and/or metadata of the processed image data, wherein the method comprises the following steps: receiving image data and/or metadata of the image data and an encrypted first hash value; decrypting the first hash value with a public key which corresponds to a first private key with which the first hash value is encrypted; generating a second hash value of the image data and/or of the metadata of the image data; comparing the first hash value with the second hash value; processing the image data; if the first hash value matches the second hash value, generating a third hash value of the processed image data and/or of the metadata of the processed image data, wherein the metadata comprises information of the processing of the image data, and encrypting the third hash value with the first private key and/or a second private key to generate a digital signature of the processed image data and/or of the metadata of the processed image data; and storing the third hash value, in particular in the metadata.

One advantage hereof is that not only the processed image/image data can digitally be signed, but the metadata of the processed image/image data can also digitally be signed. By this, much more information can be authenticated/verified later. This applies in particular if the image data has been processed/edited before digitally signing the image data by the (trusted) source/entity with the private key. The method can be carried out on the image acquiring entity and/or on a computer connected securely to the image acquiring entity. Alternatively, the method can be carried out by a reliable software which has access to the first private key. The image data can be received from the image acquiring entity which acquires the image data. Also, devices that have verified the original digital signature can digitally sign the processed image data and/or metadata of the processed image data with their private key and, thus, add a further digital signature with their private key (which can be different from the private key used to sign the original image data in the image acquiring entity). Thus, the entity which digitally signs the processed image data can be a different one as the image acquiring entity. Thus, the method is very flexible, e.g., when the image acquiring entity with which the image data was acquired and/or the original private key is not available or existing anymore. It is possible that the received image data has been processed before.

According to one embodiment of the method for authenticating image data, the image data comprises processed image data and the metadata comprises information about the processing of the image data. By this, not only unprocessed images/image data but also images/image data which have been processed by the image acquiring entity or other entities/devices can be authenticated. Furthermore, the processing can be traced back to the image data before the (last) processing step. One advantage hereof is that, even if the image data has been digitally resigned after a first digital signing, the user can trace back the authenticity to the original image data. Hence, by using the encrypted hash value of the unprocessed image data and the unprocessed image data, the user can apply the authentication method again on the encrypted hash volume of the unprocessed image data and the original image data. The user has already verified in a first step that the processed image data has been processed/edited by a reliable entity, which signed the processed image data and the corresponding metadata with its private key, and is based on a claimed original image data. Then the user can also verify that the claimed original image data has been acquired from the claimed source and with the claimed acquisition parameters which are part of the metadata. This also applies to a possible processing/editing of the image data after the image data has been acquired. One further advantage hereof is this that the acquiring of the image data including the processing/editing of the image data can be verified reliably. By this, even if the image data has been altered by a (trusted) entity, it can be proven that the image data is derived from the original unaltered image data, i.e., the image data originally acquired. In particular, it can be tested if the image data has been processed the way it is claimed in the metadata and no other processing steps have been carried out on the image data. Thus, a whole chain of processing of the image data and the metadata can be digitally signed and can be verified.

According to one embodiment of the method for authenticating image data, the positive image authenticating signal comprises the hash value of the image data and/or of the metadata before the last processing step. When the image data has been processed several times by different entities, after the first authenticating method, the next hash value is retrieved/outputted. With this retrieved/outputted hash value, the authenticating method can be repeated and so on, until the method arrives at the first digitally signed image data /metadata. This enhances the security, since even after several processing steps and digital signing by different entities, the processed image data can be traced back to the original image data.

According to one embodiment of the method for authenticating image data or of the method of digitally signing processed image data, the step of decrypting the first hash value is carried out by another entity which is not the image acquiring entity which has acquired the image data. Since the entity is not the entity which acquired the image data, the method is very flexible and can be carried out on many different entities/devices. In particular, the processing of the image data and the signing of the processed image data and/or its metadata can be carried out by a first device/entity and the authenticating is carried out by another device/entity. I.e., a microscope can acquire the image data and digitally sign the image data, but the authenticity of the image data/the digital signature can be verified by a device/entity which is not the microscope (e.g., a server which has access to the public key or public keys).

According to one embodiment of the method for authenticating image data or of the method of digitally signing processed image data, the processed image data and/or the metadata of the processed image data comprises the encrypted first hash value. By this, the previous digital signature (i.e., before the last processing step) is also input in the data which hash value is calculated and then signed (i.e., encrypted with the private key). By this, a whole chain of several processing steps from the first digital signature (e.g., of the raw image data) up to the last processing step can be signed and/or authenticated. The last digital signature authenticates the last processing step, the previous digital signature (which has been digitally signed by the last digital signature) allows to authenticate the previous processing step, and so on, up until the first digital signature to the image data which can be verified, too (this can be the raw image data or image data after a first processing, e.g., reconstruction). Thus, the whole workflow or processing chain from the original image to the current image after the last processing step can be signed and/or authenticated.

According to one embodiment of the method for authenticating image data or of the method of digitally signing processed image data, the processed image data and/or the metadata of the processed image data comprises the image data before the processing. By this, the image data before processing is immediately and directly available. So, the image data before processing is available and does not have to be retrieved from a source from somewhere. Thus, in particular when the encrypted hash value of the image data before the processing is also available, the image data before processing can be authenticated immediately afterwards without retrieving any further data (besides the public key). Thus, the whole processing chain up to the first digital signature can be verified/authenticated technically easily.

According to one embodiment of the method for authenticating image data or of the method of digitally signing processed image data, the image data has been acquired by an image acquiring entity which comprises a microscope and/or a scientific image acquiring entity and/or a medical image acquiring entity. There are many parameters when acquiring image data with a microscope and the acquisition parameters are especially important when taking image data with a microscope. The same applies to a scientific image acquiring entity and a medical image acquiring entity. Thus, many or all parameters which influenced the image data/acquiring of the image data can be digitally signed and/or verified/authenticated.

According to one embodiment of the method for authenticating image data or of the method of digitally signing processed image data, the metadata comprises at least one acquisition parameter of an image acquiring entity when taking the image data with the image acquiring entity. This way, the acquisition parameters are available and can be altered to acquire new image data with the same image acquiring entity. Thus, several images/different image data with identical or similar acquisition parameters can be acquired. Alternatively or additionally, a further image data with another image acquiring entity can be taken with the same acquisition parameters.

According to one embodiment of the method for authenticating image data or of the method of digitally signing processed image data, wherein the image data is received by loading the image data from a data storage. By this, the image data can be received from the image acquiring entity or from a hard disk or a cloud based system. This enhances the possibilities of the method.

According to one embodiment of the method for authenticating image data or of the method of digitally signing processed image data, the image data and/or the metadata comprises a directory of files, in particular with subdirectories. The whole directory of the image/image data and/or the metadata can be hashed and later be authenticated. Thus, if the image data is not saved in a file but in a directory of nested sub-directories and files or in a hierarchical structure of files which could be stored in the cloud (e.g. OME-Zarr format, OME-NGFF, OME-TIFF, Hierarchical Data Format (for example, HDF5), N5-formt) the whole directory/set of files is hashed. By this, complex images/image data and/or metadata can be used for the image data and/or metadata.

According to one embodiment of the method for authenticating image data or of the method of digitally signing processed image data, the method is carried out on a server upon a request by a client and the public key corresponding to the private key is stored in a database of a server. By this, the method can be carried out by a client-server-architecture.

According to one embodiment of the method for authenticating image data or of the method of digitally signing processed image data, the first hash value is encrypted with a private key which is assigned to only one image acquiring entity which has acquired the image data and no other image acquiring entity and/or with a private key which is assigned to only one user and no other user. By this, the specific image acquiring entity or microscope with which the image data was acquired can be identified and can be authenticated. The key needs to be kept secret, i.e., that no third party has access to the private key. This improves the reliability, since even entities/microscopes with are identical in construction can create different images/different image data from the same sample with the same acquisition parameters due to manufacturing tolerances, damages etc.

According to one embodiment of the method for authenticating image data or of the method of digitally signing processed image data, the image data and its metadata are stored separately and the image data and its metadata are linked uniquely. Since the metadata can be huge, a separate storage of the metadata makes the usage of the available storage and/or bandwidth more efficient. Nevertheless, the metadata and the image/image data are referred to one another securely and unambiguously/uniquely. This can be done for example by including the hash value of either the image data or the metadata when computing the hash value of the other part (i.e., the metadata or the image data). Hence, it can be verified that the image data and the metadata belong/correspond to each other.

According to one embodiment of the method for authenticating image data or of the method of digitally signing processed image data, an error signal is generated if the first hash value does not match the second hash value. By this, a negative response is given, if the hash values do not match. This increases the security, since the user and/or client can be sure that the verifying process has been carried out.

According to one embodiment of the method for authenticating image data or of the method of digitally signing processed image data, the processed image data is a particular clipping and/or view and/or cross-section of the image data. By this, not only changes of the content of the image data can be considered but other kinds of changes of the image data are considered. This is very relevant since just clipping/changing the view or cross-section can produce a very different interpretation of the content of the image data. Thus, the reliability is increased.

According to one embodiment of the method for authenticating image data or of the method of digitally signing processed image data, the metadata comprises at least one table with information about the image data and/or the image content and/or the metadata comprises a watermark and/or a QR-code and/or the metadata comprises quantitative analysis data of the image data, e.g., information about the cell segmentation in the image data and/or the number of cells in the image data. One advantage hereof is that the at least one table can be digitally signed/authenticated, too. Thus, one can trust that the at least one table and its content was created by a reliable source. This saves time, since the at least one table does not have to be created/recreated, but can be used as a start for further analysis of the image data/image content. One advantage hereof is that further information about the image data is also digitally signed. This saves time, since authenticated information and/or analysis data is also available and can be used instead of acquiring the information again or creating the analysis data again. Furthermore, by having a watermark, the authenticity of the image data can be verified in another way additionally. Further, via the QR-code a direct/unique link to the image data can be stored in the metadata.

The problem is also solved by a computer program product having instructions which are readable by a processor of a computer and which, when they are executed by the processor, cause the processor to carry out the method as described above.

The problem is also solved by a computer-readable medium storing the computer program product described above.

The image acquiring entity can comprise or be a physical device (e.g., a microscope), a computer and/or a software.

The "entity" which is different from the image acquiring entity can comprise or be another physical device or can be or comprise software which is not the software with which the image data was acquired/generated originally.

The respective hash value can be calculated with SHA-1, SHA-2 (e.g., SHA-256) or SHA-3 or another hashing function. The image data and/or metadata can be stored on a hard disk or in a cloud based system.

The public key and the private key can be part of a set of keys of the RSA-algorithm.

The image data and/or the metadata can comprise one or more of the following:
General metadata
   - the category of the image (e.g., raw image or processed image)
   - the digital signature of the image data from which the current image data has been processed
   - annotation and labels of the image to be used to train a machine-model and/or a deep learning model
Sample related data
   - sample preparation steps, sample IDs, barcodes, QR codes
   - sample carrier information (plate and well information, slide labels, preview images, sample substrate: cover glass type and thickness, material etc.)
Acquisition metadata
   - image data acquisition settings/parameters
      frame rate, sampling rate, Z-Spacing, Dimension Order, TileRegions, Positions, Position Arrays, Focus Points
      all light source parameters, such as: intensity, wavelength, voltage, current, spectrum, used spectral emission and detection windows, scanner, channel and z-Stack alignment, transformations between different imaging modalities used during image acquisition
      all detector settings, such as: exposure time or dwell time, analog and digital gains, binning, scan speed, readout Modes
      markers from external signals (TTL, Trigger Pulses)
      magnification, numerical aperture, zoom level, additional optical elements
      environmental and incubation settings
   - versions of used acquisition software and operating system
   - UserIDs, date & time of creation
   - microscope hardware specifications
      model and vendor of the microscope
      optical parameters of imaging system incl. filter specifications
      metrics for instrument performance
      calibration files (white balance, shading, point spread functions, beam offsets, scanner
      firmware version
   - image data Information
      image data dimensions (hypercube)
      image data pixel scaling and resolution levels (pyramidal levels)
      pixel types(s)
      file size (compressed or uncompressed)
      compression methods
      channel structure
      stage axis parameters (e.g., 6 axis parameters), in particular XYZ stage positions
Image data reconstruction / processing metadata
- pre- and postprocessing algorithms and parameters such as
   filtering
   deconvolution
   denoising
   stitching
   airycsan processing,
   SIM and SLMM processing
   Al-based Image-to-Image transformations
   contrast enhancements
   shading corrections
   bleaching corrections
   drift corrections
- image data transformations and registration parameters and coordinate systems
- display settings such as
   colormaps
   gamma
   min and max values
- additional image layers (labels and annotations)
- image data analysis parameters
   typically XML-like descriptions of used analysis pipeline
- unique IDs of used Machine-Learning or Deep Leaning models
- audit trail of applied processing function
- image data analysis results
   tabular data
   objects (label images, tabular data or structured data)

In addition to the previously described metadata, the metadata can also comprise attachments: E.g., annotations, preview or label images, barcodes, tabular data and custom data with application-specific formats.

The hash algorithm can be kept secret, e.g., in the image acquiring device and/or in the computer.

The encryption/decryption of the hash value can be a symmetric encryption, e.g., Advanced Encryption Standard (AES). By this, the public key and the private key are identical and the public key should be kept secret, too. Alternatively hereto, the public key and the private key belong to a key pair/set, wherein the private key cannot be calculated in a reasonable amount of time from the public key, e.g, the public key and private key are a key pair of the RSA-algorithm.

The term "digitally signing" can comprise creating the hash value with a hash algorithm and encrypting the hash value with a secret private key while the public key corresponding to the secret key is stored separately from the private key.

The "processing" of the image data can comprise any form of alteration of the image data and/or analyzing the image data. The processing of the image data can comprise or be any kind of editing/transforming/changing/analyzing the image data. Processing can include, for example, changing the contrast of the image data or a part of the image data, changing the colors of a part of the image data or the whole image data, denoising, deblurring, reconstructing the image data or part of it and/or removing artifacts of the image data of a part of the image data. The processing can comprises reconstructing image data from image data such that the processed/reconstructed image data is interpretable by a human (for example reconstructing a 3D volumetric image from 2D projections in x-ray/computer tomography, or reconstructing a high resolution image from a set of low resolution images in Airyscan or structured-illumination microscopy). The processing can have more than one image or more data than from one image acquiring process as input and can output one image/image data or several images or a multitude of image data.

The processed image data can be or comprise image data which has been changed and/or data which has been generated by analyzing the image data (e.g., object parameters etc.). I.e., the image data does not have to be changed when processing the image data, but the processed image data can comprise qualitative and/or quantitative analysis/information about the image data and/or the content of the image data.

Preferred embodiments are evident from the dependent claims. The invention is explained in more detail below with reference to drawings of exemplary embodiments. In the figures:
- Fig. 1: shows a schematic view of the microscope, the computer, the image data and the metadata;
- Fig. 2: shows a schematic flowchart of the method of digitally signing image data and/or its metadata;
- Fig. 3: shows a schematic flowchart of the method for authenticating an image data and/or its metadata; and
- Fig. 4: shows a schematic view of a server and a client.

The same reference numerals are used in the following description for identical parts and parts having an identical effect.

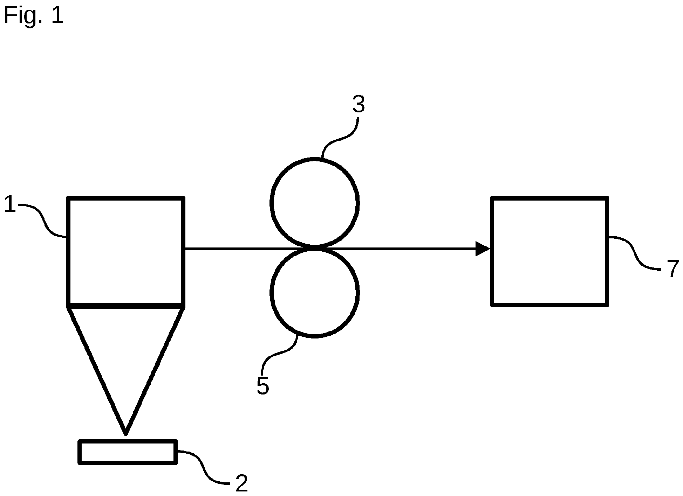

Fig. 1 shows a schematic view of a microscope 1, a computer 7, image data 3 acquired by the microscope and metadata 5 of the image data 3.

The image acquiring entity can comprise or be a microscope 1. In Fig. 1, the entity is a microscope 1 which acquires image data 3 of a sample 2. The sample 2 can be a sample of a living being (e.g., bacteria and/or viruses) and/or of dead material (e.g., a rock).

The image data 3 can comprise or be a single image, e.g., taken with a camera. Also, it is possible that the image data 3 comprises information about the sample which cannot be interpreted by a human and/or be recognized by a human, i.e., a human cannot comprehend the information of the image data directly. The image data 3 can be reconstructed/processed to create an image/image data interpretable by a human directly.

The image data 3 can comprise a multitude of images/image information of the sample, e.g., taken from different angles/perspectives. E.g., the image data 3 is/the images acquired by a computer tomography device. The single image/image data is not interpretable by a human. The image data 3 is reconstructed by software or a computer (e.g., by combining several images) to create an image or multiple images interpretable by a human. Thus, then the reconstructed image data can be an "image", i.e., optical information understandable by a human.

The image data can be acquired with visible light or (for the human eye) invisible radiation (e.g., infrared, ultra-violet, X-ray etc.).

The microscope 1 can be connected to a computer 7/software and/or controlled by a computer/software.

Initially, in a first step 19 a set of a private key and a public key 11 of a cryptographic algorithm is generated for the microscope 1 hardware and/or for the system of microscope 1 and the computer 7 controlling the microscope 1 and/or for the computer 7 controlling the microscope 1 and/or for a user. The private key is held secret. The cryptographic algorithm can be the RSA-algorithm. The private key can be stored on a smart card or on the microscope 1 and/or computer 7. The private and public key 11 can be generated for example by the manufacturer of the microscope 1 at the factory, before the instrument is shipped or it can be generated upon the registration of the microscope 1 and/or microscope 1/computer-system and/or computer/software-system and/or a user. It is possible that the private key is accessible through a password protected application for a registered user and/or by means of a smartcard interface.

The public key 11 is available to a server 9 and stored, e.g., in a database 10.

The private key can be different for every microscope 1 and/or user and/or software, e.g., every microscope 1 has its own unique private key. Alternatively, it is possible that the private key is the same for several microscopes and/or users and/or software. I.e., several microscopes (e.g., from the same manufacturer) and/or users and/or software share the same key.

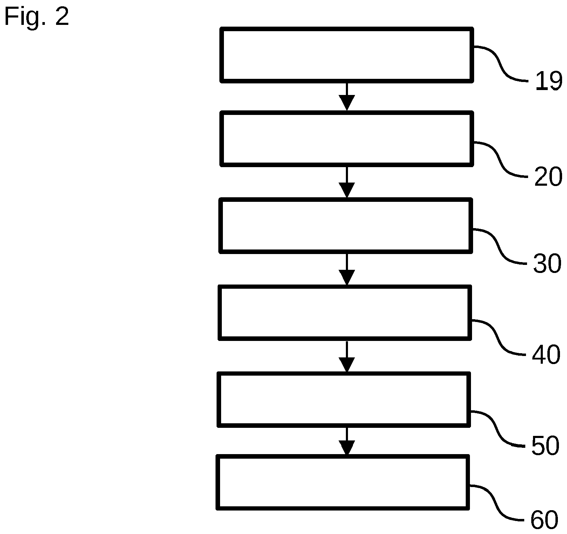

Fig. 2 shows a schematic flowchart of the method of digitally signing an image data 3 acquired by the microscope 1 and/or its metadata 5. The method can be carried out by the microscope/computer-system which acquired the image data 3 or by a computer system/software which has access to the private key.

When (raw) image data has been acquired by the microscope 1 or by the computer 7 with the microscope 1, the image/the image data and its metadata 5 (e.g., the acquisition parameter) are hashed. I.e., a first hash value of the image (data) and the metadata 5, which belongs/corresponds to the image data 3, is generated. The metadata 5 can comprise information about the parameters of the acquisition process of the image data 3 (focus information, shutter opening time information etc.), information about the sample 2 etc.

The image/image data and/or metadata 5 can comprise several files and/or can comprise one or more directories of files. The hashing can be done by iteratively hashing the individual files, and then hashing the set of hashes corresponding to a sub-directory / hierarchy level. The steps are repeated iteratively from the lower levels of hierarchy to the highest until a single hash is returned. The files can be stored locally, in the network and/or in a cloud-based system. E.g., a fraction of the files of the image data and/or corresponding metadata can be stored locally while the remaining fraction is stored in a cloud-based system. Each file is or several files together are hashed (i.e., a hash value is calculated), then the files in the next directory (locally, in the network and/or in the cloud-based system) are hashed and so on. Afterwards, a hash value of the hash values is calculated. If necessary, the last step can be repeated until one single hash value is calculated/generated for the whole directory (with subdirectories). I.e., the single hash value at the end can be the hash value of other hash values, wherein each hash value is again the hash value of several other hash values. The number of levels in the hierarchy of the image data and/or the metadata corresponding to the image data can be 1, 2, 3, 4 or more than 4. Also, more than one directory can exist at each level of the hierarchy, wherein all directories at the same level are considered and hashed.

The hash values can be encrypted (with the private key) before the hash value is hashed with other hash values together. Alternatively, the hash values can be unencrypted, wherein the last/final hash value is digitally signed, i.e., encrypted, with the private key.

At the end, one first hash value is generated/calculated. The first hash value is a digital signature of all files and/or directories of the image data and/or the metadata.

The first hash value is encrypted with the private key of the microscope/the microscope-computer-system and/or the user and is stored. By this, the first hash value is digitally signed. The digital signature is a digital signature of all hash values which were used for calculating the first hash value.

The digital signature can be verified by decrypting the first hash value with the public key 11 (step 30), which corresponds to the private key, and comparing the decrypted first hash value with a hash value generated from the image/image data and its metadata 5/the corresponding metadata 5 (without the first hash value in the metadata 5 and/or image data 3) (step 30, step 40 and step 50). If the first hash value matches the generated hash value, the digital signature has been verified successfully. If the first hash value does not match the generated hash value, the digital signature, at least with this public key 11, is not verified successfully. In the latter case, further other public keys 11 can be used for trying to verify the digital signature. If the public key 11 or none of the available public keys 11 leads to a successful verification of the digital signature, an error message/negative image authenticating signal 16 can be generated (step 60).

The image data 3, its metadata 5 and the encrypted first hash value/digital signature can be stored separately from each other or together. If the image data 3, its metadata 5 and the encrypted first hash value are stored separately, a unique link between the image data 3, its metadata 5 and the encrypted first hash value can be stored in the image data 3, its metadata 5 and/or in the first hash value (before encrypting it).

The encrypted hash value is the digital signature of the image/image data and/or its corresponding metadata 5. The digital signature can be attached to the image data and/or the metadata 5. Additionally or alternatively, the digital signature can be stored in the name of the image file and/or in the name of the metadata 5 files and/or in the name of the directory/directories. Another possibility is to store the digital signature separately from the image/image data 3 and the metadata 5.

The image data 3, the metadata 5 and the encrypted hash value can be stored on a hard disk or in a cloud based system. The public key(s) 11 can be stored on the server 9.

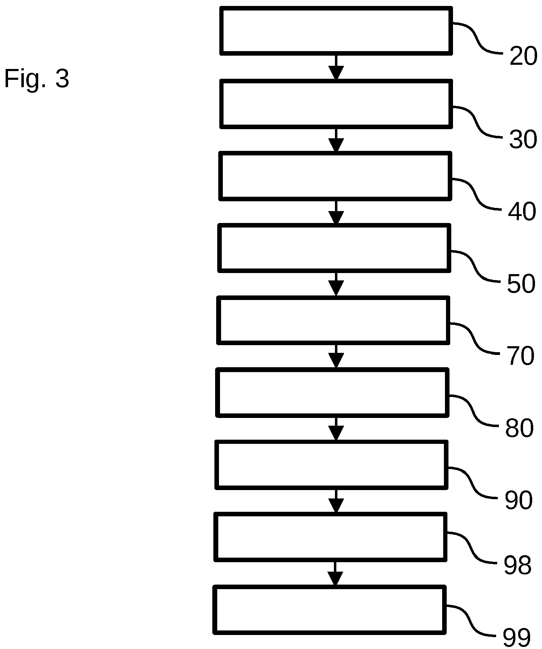

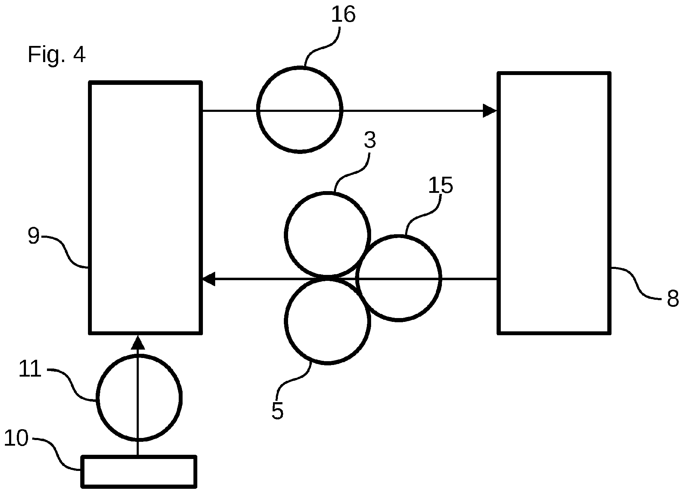

Fig. 3 shows a schematic flowchart of the method for authenticating an image data 3 and/or its metadata 5. Fig. 4 shows a schematic view of a server 9 and a client 8.

First, in a step 20, the image data 3 and the metadata 5 which belongs/corresponds to the image data 3 as well as the encrypted first hash value is received. The data or part of the data (e.g., the image data 3 and its corresponding metadata 5 and the encrypted first hash value 15) can be sent from a client 8 to a server 9 and the method can be carried out on the server 9. The encrypted first hash value/digital signature can be part of the metadata 5 and/or the image data 3. The metadata 5 and/or the image data 3 is searched for an encrypted hash value/digital signature. When the metadata 5 comprises a digital signature, the digital signature can be removed (temporarily) from the metadata 5.

Then, the image/image data and metadata 5 is hashed (with the same hashing algorithm which was used for creating the digital signature), i.e., a second hash value is generated (step 40).

One or all public keys 11 available (e.g., in a database 10 of the server 9 or accessible from the server 9 and/or received from the client 8) will be used to try to decrypt the encrypted first hash value. One or each one of all decrypted hash values will be compared to the second hash value (step 50). If any one of the (decrypted) hash values matches/is identical to the generated second hash value, a positive image authenticating signal 16 is generated (step 60). This can be done separately for a hash value of the image/image data and for a hash value for the metadata 5, wherein a permanent unique and verifiable link between the image/image data and the metadata 5 is present. If one or none of the decrypted hash values matches to the generated second hash value, a negative image authenticating signal 16 is generated (step 60).

The positive or negative image authenticating signal 16 can be sent to the client 8 (see Fig. 4). A positive image authenticating signal 16 means that the image data and its metadata 5 are original/authentic/verified successfully, i.e., the digital signature is valid. Hence, the image data/metadata 5 belongs to the claimed image data and the image data has been unaltered (or has been only altered in the way described in the metadata 5).

The authenticating method can be carried out by an entity which is not the entity which acquired and/or signed the original image data, i.e., which created the first digital signature for the image data and/or its metadata 5. E.g., a server 9 authenticates the image data 3 and its metadata 5.

Instead of or additionally to digitally signing the raw image data/unprocessed image data, the processed image data can be digitally signed. For this, either the raw image data and its corresponding metadata 5 has to be authenticated before or directly after acquiring the image data 3 with the microscope 1, the image data 3 is processed (in a secure environment) and then the processed image data and its metadata 5 are digitally signed. The metadata 5 can comprise category metadata 5 (namely, if the image data is a processed image data or raw image data), the parent file digital signature (containing the digital signature of the raw image dataset if this was loaded/authenticated before), sample meta-data, acquisition meta-data and/or processing meta-data, etc.

The parent file digital signature can comprise the encrypted hash value of the image data and/or the metadata before the present/last processing step. This digital signature can be part of the processed image data and/or of the metadata of the processed image data.

When authenticating a processed image data, the authentication process can be recursive. This means that first the digital signature of the processed image data and its metadata 5 is verified. Then, a second digital signature (also called parent file digital signature) within the image data 3 or metadata 5 (which is different from the first digital signature) is searched and removed from the image data/metadata 5. Then, the second digital signature is verified/authenticated (by computing the hash value of the image data 3 and its metadata 5 without the second digital signature and comparing the computed hash value of the image data 3 and/or its metadata 5 without the second digital signature with the decrypted hash value of the second digital signature). Afterwards, if the second digital signature has been verified, a third digital signature is searched and, if found, then verified/authenticated (by computing the hash value of the image data 3 and its metadata 5 without the third digital signature and comparing the computed hash value with the decrypted hash value of the third digital signature). This can be done iteratively, until no further digital signature is found in the metadata 5 or associated with the image data 3.

By this, when the image data 3 has been processed/edited several times, one process step after the other and one processed image data after the other is verified/authenticated up until the original image data which has been acquired by the microscope 1 or the first digitally signed image data by the microscope 1 and/or user. This way, the whole chain of digital signatures up until the original image data can be traced back and verified/authenticated, wherein the digital signature of each processing step and each processed image data is interconnected with the previous processing step and with the image data before the previous processing step. If all verifying/authenticating steps are successful, a positive image authenticating signal 16 can be generated. Otherwise, a negative image authenticating signal 16 can be generated.

The respective data of the parent image data and/or parent metadata should be available for the recursive authentication process.

The different verifying steps can be carried out by a single server 9. The different digital signatures can be done with different private keys, if all corresponding public keys 11 are available.

Also, by this, at each verifying step, alternative processing of the image data 3 can be carried out and then the newly processed image data with the metadata 5 (comprising all processing steps until then and the new processing step as well as the digital signature of the image data before the latest/newest processing step) can be resigned with the private key.

Also, the raw image data can be retrieved and the workflow/processing chain from the raw image data to the image data after the last/most recent processing step, i.e., the last/most recent digital signature, can be authenticated. By this, any of the retrieved processing steps can be repeated with adjusted/changed parameters without data repetition.

If the verification of a loaded/received image data and/or its metadata 5 is not successful, no resigning of the (processed) image data and/or its metadata 5 with the private key is possible.

The verification and digital signing of the (newly) processed image data can be carried out by an entity, e.g., a computer and/or software, which is different from the entity, e.g., microscope 1 and/or computer and/or software, which has digitally signed the original unprocessed image data or which has made the first digital signature to the processed image data after acquiring the image data.

Usually, the software only allows (re)signing a processed image data and/or its metadata after authenticating the image and/or its metadata before the processing. Then, only the present processed image data and/or its metadata needs to be authenticated since one trusts the software in that only authenticated image data and/or metadata may be signed, i.e., the image data and/or the metadata has been authenticated before the (most recent) signature.

It is also possible, that the image data has been processed by different entities, e.g., different computers and/or microscopes. Then, as a last step of the authenticating/verifying on one computer, the parent file digital signature can be outputted. With this, the user and/or client 8 can make a request to another entity with this digital signature and the image data/image data and its metadata 5 at this stage to verify/authenticate this digital signature with another entity.

E.g., a first microscope/computer/server/software can verify/authenticate the latest 2 steps of editing/processing the image data, but the previous 3 processing steps have been done by a second computer, which public key is not available to the first microscope/computer/server/software. Thus, in the original verification request, there are 2 digital signatures of the first computer which can be authenticated by the first server or first computer or with the public key of the first computer/microscope. Afterwards, the metadata 5 after removing the 2 digital signatures and the image data before these 2 processing steps is outputted by the request/by the first microscope/computer/server/software. The user/client 8 can then use these data to make another request to a second microscope/computer/server/software to verify/authenticate the remaining 3 digital signatures. When the 2 digital signatures have been verified and the 3 digital signatures have been verified successfully, the present image data and its metadata 5 is verified as a whole.

The processed image data can comprise a single "view"/perspective of the image/image data and/or a "snapshot"/cross-section of the image/image data (e.g. an image corresponding to a certain z-level and/or a x-y crop of a 3D dataset) and/or a clipping of a certain section of the image/image data. The metadata 5 can comprise information about the view/perspective/snapshot/cross-section, in particular how this is generated from the original image data and/or a previously processed image data. The snapshot/cross-section/image/cropped image can be an image or image data of a publication (e.g., a scientific paper). The digital signature(s) of the respective snapshot/cross-section/image/cropped image can be published with the publication (e.g., via a QR-code), so that every reader can verify its authenticity. This can be done up to the original image data/the first digital signature in a chain of digital signatures.

It is also possible to append a watermark and/or a QR-code to the image data and/or its metadata 5 before and/or after digitally signing the image data 3 and its metadata 5. The QR-code can provide further information about the image data 3, its metadata 5 and/or the digital signature. In particular, the watermark can make a link between the image data 3 and its metadata 5 or the place where its metadata 5 is stored.

The metadata can comprise information about the several processing steps, i.e., a workflow, which has been performed on the image data. For example, the metadata can comprise information about a deep-learning model, e.g., the identification number of the respective deep-learning model used in the processing step.

In a further embodiment, the metadata 5 can comprise quantitative analysis data, e.g., tabular data, based on the image data 3 (e.g. cell segmentation and counting, etc.).

The server 9 has access to a database 10 that contains many or all public keys 11 corresponding to all registered users and/or microscopes or scientific imaging hardware.

The image data 3 and its metadata 5 can be stored in the Carl Zeiss Image-format (.czi; cf. https://www.zeiss.com/microscopy/en/products/software/zeiss-zen/czi-image-file-format.html), in the TIFF-format (.tiff), in the OME-Zarr-format, OME-NGFF-format (cf. Moore, J. (2021). "OME-NGFF: a next-generation file format for expanding bioimaging data-access strategies" in Nature Methods, 18, 1496-1498. doi:10.1038/s41592-021-01326-w) or in the JPEG-format (.jpg) or PNG-format (.png) or Single-Image-Stack-format (.sis) or another image format.

The (processed) image/image data and the metadata 5 can be digitally signed together or individually. I.e., one hash value of both the image/image data and the metadata 5 is generated or one hash value for the image/image data and one further hash value for the metadata 5 is generated. In the latter case, the image/image data and the metadata 5 can comprise a permanent unique link which refers to the corresponding metadata 5 or the corresponding image/image data.

The image/image data can comprise an image dataset, i.e., a set or bundle of several images. The metadata 5 can comprise metadata 5 acquired from different entities/systems that were correlated when acquiring the image data. The correlation can be established by a registration method. The image/image data can include multiple datasets and the description of the used transformation which ensures the correct alignment which is required to get valid results in subsequent processing steps. This means, for example, that the image acquiring entity can comprise several subsystems, e.g., a light source and a microscope 1. The metadata 5 can comprise the correlation, e.g., relative position to each other, of the several subsystems. By this, all information about the acquiring of the image data/images (i.e., information about all subsystems) are stored in the metadata 5 which are necessary to recreate the exact same image data/images.

After each step of a workflow of the image data, the image data and/or its metadata can be digitally signed. E.g., first, the image data is acquired, then the image data is reconstructed/processed, then the image data is further processed and next quantitative analysis of the image data and/or its metadata is carried out. After each step, the image data at this stage and/or its metadata is hashed and encrypted with the private key as a digital signature. The previous digital signatures and/or the image data before each step can be kept in the image data and/or in the metadata before the next signing step.

### Reference numerals

- 1: image acquiring entity/microscope
- 2: sample
- 3: image data
- 5: metadata
- 7: computer
- 8: client
- 9: server
- 10: database
- 11: public key
- 15: encrypted first hash value
- 16: positive or negative image authenticating signal
- 19: step of generating the public and private key set
- 20: step of receiving the image
- 30: step of decrypting the first hash value
- 40: step of generating a second hash value
- 50: step of comparing the first hash value with the second hash value
- 60: step of outputting a positive or negative image authenticating signal
- 70: step of processing the image data
- 80: step of generating a third hash
- 90: step of encrypting the third hash value
- 98: step of storing the third hash value
- 99: step of encrypting the third hash value with the private key to generate a digital signature of the processed image data

## Claims

1. Method for authenticating image data (3) and/or metadata (5) of the image data (3), wherein the method comprises the following steps:
receiving the image data (3) and/or the metadata (5) and an encrypted first hash value of the image data (3) and/or the metadata (20), wherein the metadata (5) comprises information about the image data (3);
decrypting the first hash value with a public key (11) (30), wherein the public key (11) corresponds to a private key with which the first hash value is encrypted;
generating a second hash value of the image data (3) and/or the metadata (5) (40);
comparing the decrypted first hash value with the second hash value (50); and
generating a positive image authenticating signal (16) if the first hash value matches the second hash value or a negative image authenticating signal (16) if the first hash value does not match the second hash value (60).

2. Method according to claim 1, wherein
the positive image authenticating signal (16) comprises the hash value of the image data (3) and/or of the metadata (5) before the last processing step.

3. Method of digitally signing processed image data (3) and/or metadata (5) of the processed image data (3), wherein the method comprises the following steps:
receiving image data (3) and/or metadata (5) of the image data and an encrypted first hash value (20);
decrypting the first hash value with a public key (11) which corresponds to a first private key with which the first hash value is encrypted (30);
generating a second hash value of the image data (3) and/or of the metadata of the image data (5) (40);
comparing the first hash value with the second hash value (50);
processing the image data (3) (70);
if the first hash value matches the second hash value, generating a third hash value of the processed image data and/or of the metadata (5) of the processed image data (98), wherein the metadata (5) comprises information of the processing of the image data, and encrypting the third hash value with the first private key and/or a second private key to generate a digital signature of the processed image data and/or of the metadata of the processed image data (99); and
storing the third hash value, in particular in the metadata (5).

4. Method according to any of the previous claims, wherein
the step of decrypting the first hash value is carried out by another entity which is not the image acquiring entity (1) which has acquired the image data.

5. Method according to any of the previous claims, wherein
the processed image data and/or the metadata (5) of the processed image data comprises the encrypted first hash value.

6. Method according to any of the previous claims, wherein
the processed image data and/or the metadata (5) of the processed image data comprises the image data before the processing.

7. Method according to any of the previous claims, wherein
the image data has been acquired by an image acquiring entity (1) which comprises a microscope (1) and/or a scientific image acquiring entity and/or a medical image acquiring entity.

8. Method according to any of the previous claims, wherein
the metadata (5) comprises at least one acquisition parameter of an image acquiring entity (1) when taking the image data (3) with the image acquiring entity (1).

9. Method according to any of the previous claims, wherein
the image data (3) and/or the metadata (5) comprises a directory of files, in particular with subdirectories.

10. Method according to any of the previous claims, wherein
the first hash value is encrypted with a private key which is assigned to only one image acquiring entity (1) which has acquired the image data and no other image acquiring entity (1) and/or with a private key which is assigned to only one user and no other user.

11. Method according to any of the previous claims, wherein
the image data (3) and its metadata (5) are stored separately and the image data (3) and its metadata (5) are linked uniquely.

12. Method according to any of the previous claims, wherein
the processed image data is a particular clipping and/or view and/or cross-section of the image data (3).

13. Method according to any of the previous claims, wherein
the metadata (5) comprises at least one table with information about the image data (3) and/or the image data content and/or the metadata (5) comprises a watermark and/or a QR-code and/or the metadata (5) comprises quantitative analysis data of the image data (3), e.g., information about the cell segmentation in the image data (3) and/or the number of cells in the image data (3).

14. Computer program product having instructions which are readable by a processor of a computer and which, when they are executed by the processor, cause the processor to carry out the method as claimed in any of the preceding claims.

15. Computer-readable medium storing the computer program product as claimed in claim 14.
